(19) Europäisches Patentamt
European Patent Office
Office européen des brevets

(11) Veröffentlichungsnummer: **0 518 388 A2**

# (12) EUROPÄISCHE PATENTANMELDUNG

(21) Anmeldenummer: **92114477.0**

(51) Int. Cl.5: **A61K 31/225**

(22) Anmeldetag: **20.06.88**

Diese Anmeldung is am 25 - 08 - 1992 als Teilanmeldung zu der unter INID-Kode 60 erwähnten Anmeldung eingereicht worden.

(30) Priorität: **19.10.87 US 109780**

(43) Veröffentlichungstag der Anmeldung: **16.12.92 Patentblatt 92/51**

(60) Veröffentlichungsnummer der früheren Anmeldung nach Art. 76 EPÜ: **0 312 697**

(84) Benannte Vertragsstaaten: **AT BE CH DE ES FR GB GR IT LI LU NL SE**

(71) Anmelder: **Speiser, Peter Paul, Prof. Dr.**
**Freudenbergstrasse 101/D2**
**CH-8044 Zürich(CH)**
Anmelder: **Joshi, Rajendra K., Dr.**
**Altstetterstrasse 224**
**CH-8048 Zürich(CH)**

(72) Erfinder: **Speiser, Peter Paul, Prof. Dr.**
**Freudenbergstrasse 101/D2**
**CH-8044 Zürich(CH)**
Erfinder: **Joshi, Rajendra K., Dr.**
**Altstetterstrasse 224**
**CH-8048 Zürich(CH)**

(74) Vertreter: **Schwabe - Sandmair - Marx**
**Stuntzstrasse 16**
**W-8000 München 80(DE)**

## (54) Pharmazeutische Zubereitungen zur Behandlung der Psoriasis, psoriatischen Arthritis, Neurodermitis und Enteritis regionalis Crohn.

(57) Die Erfindung betrifft pharmazeutische Zubereitungen zur Behandlung der Psoriasis und psoriatischen Arthritis, Neurodermitis und von Enteritis regionalis Crohn, enthaltend eine oder mehrere Verbindungen aus der Gruppe der Calcium-, Magnesium-, Zink- und Eisensalze von Fumarsäure-Monoalklylester der allgemeinen Formel

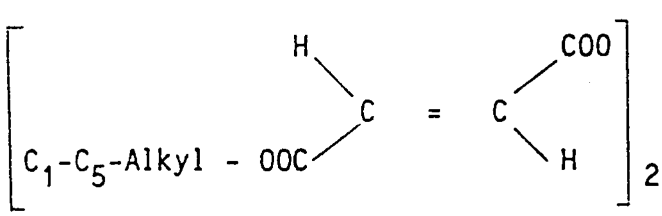

Ca(Mg, Zn, Fe),

gegebenenfalls im Gemisch mit Dialkylfumarat der Formel

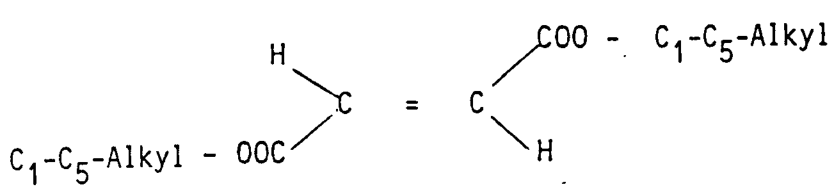

und üblichen pharmazeutischen Hilfs- und Trägerstoffen.

EP 0 518 388 A2

Die Erfindung betrifft pharmazeutische Zubereitungen zur Behandlung der Psoriasis , psoriatischen Arthritis, Neurodermitis und Enteritis regionalis Crohn (Morbus Crohn) zur systemischen Therapie dieser Krankheiten.

Pharmazeutische Zubereitungen, die nach Verabreichung bei ihrem biologischen Abbau in den Zitronensäurezyklus einmünden oder diesem angehören, gewinnen meist in hoher Dosierung immer mehr an therapeutischem Wert, da man mit ihrer Hilfe kryptogenetisch bedingte Krankheiten zu lindern oder zu heilen vermag.

So hemmt Fumarsäure das Wachstum des Ehrlich-Aszittumors bei Mäusen, vermindert die toxischen Effekte von Mitomycin C und Aflotoxin (K. Kuroda, M. Akao, Biochem. Pharmacol. 29, 2839-2844 (1980) / Gann. 72, 777-782 (1981) / Cancer Res. 36, 1900-1903 (1976)) und besitzt eine antipsoriatische sowie antimikrobielle Wirkung (C. N. Huhtsnen, J. Food Sci. 48, 1574 (1983) / M. N. Islam, U.S.-Patent 4 346 118 vom 24. August 1982 / C. A. 97, 161317b (1982)).

Hohe Verabreichungsdosen von Fumarsäure oder ihrer bisher bekannten Derivate wie Dihydroxyfumarsäure, Fumaramid und Fumaronitril besitzen bei parenteraler, dermaler, insbesondere aber peroraler Verabreichung eine derart unzumutbare Nebenwirkungsrate und hohe Toxizität (P. Holland, R. G. White, Brit. J. Dermatol. 85, 259-263 (1971) / M. Hagedorn, K. W. Kalkoff, G. Kiefer, D. Baron, J. Hug, J. Petres, Arch. Derm. Res. 254, 67-73 (1975)), daß bisher meist von einer solchen Therapie abgesehen werden mußte.

In der europäischen Patentanmeldung Nr. 85 116 011.9 vom 16. Dezember 1985 sind bereits Fumarsäurederivate und sie enthaltende pharmazeutische Zubereitungen zur Behandlung der Psoriasis beschrieben. Es wurde nunmehr überraschend gefunden, daß eine insgesamt erheblich verbesserte Wirkung durch Mischpräparate erzielt werden kann, die eine oder mehrere Verbindungen aus der Gruppe der Calcium-, Magnesium-, Zink- und Eisensalze von FumarsäureMonoalkylester der allgemeinen Formel

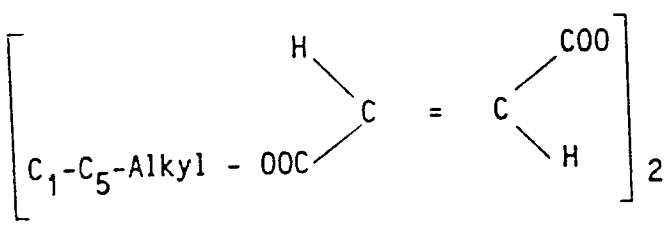 Ca(Mg, Zn, Fe),

allein oder vorzugsweise im Gemisch mit Dialkylfumarat der Formel

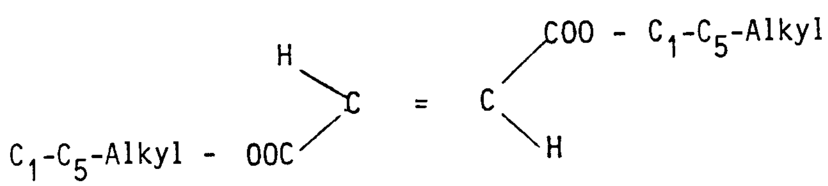

und üblichen pharmazeutisch verträglichen Hilfs- und Trägerstoffe enthalten.

Bevorzugte Mischpräparate gemäß der Erfindung enthalten das Calciumsalz des Fumarsäure-Monoethylesters, das Calciumsalz des Fumarsäure-Monoethylesters im Gemisch mit Dimethylfumarat, das Calcium- und Zinksalz des Fumarsäure-Monoethylesters im Gemisch mit Dimethylfumarat oder das Calcium-, Magnesium- und Zinksalz des Fumarsäure-Monoethylesters im Gemisch mit Dimethylfumarat.

Zur oralen Verabreichung sind besonders Mischpräparate geeignet, die das Calciumsalz des Fumarsäure-Monoalkylesters in einer Menge von 100 bis 300 mg enthalten, wobei das Gesamtgewicht der Wirkstoffe 100 bis 300 mg beträgt.

Weitere bevorzugte orale Verabreichungsformen enthalten 10 bis 290 Gew.-Teile des Calciumsalzes des Fumarsäure-Monoalkylesters und 290 bis 10 Gew.-Teile Dimethylfumarat, 10 bis 250 Gew.-Teile des Calciumsalzes des Fumarsäure-Monoalkylesters, 1 bis 50 Gew.-Teile Dimethylfumarat und 1 bis 50 Gew.-Teile des Zinksalzes des Fumarsäure-Monoalkylesters oder 10 bis 250 Gew.-Teile des Calciumsalzes des Fumarsäure-Monoalkylesters, 250 bois 10 Gew.-Teile Dimethylfumarat, 1 bis 50 Gew.-Teile des Magnesiumsalzes des Fumarsäure-Monoalkylesters und 1 bis 50 Gew.-Teile des Zinksalzes des Fumarsäure-Monoalkylesters, wobei jeweils das Gesamtgewicht der Wirkstoffe 100 bis 300 mg beträgt.

Für den systemischen Einstieg in die Behandlung bzw. den Ausstieg ist eine niedrige Dosierung vorteilhaft, die beispielsweise aus 30,0 mg Dimethylfumarat, 67,0 mg des Calciumsalzes von Monoethylfumarat, 5,0 mg des Magnesiumsalzes von Monoethylfumarat und 3,0 mg des Zinksalzes von Monoethylfumarat enthält.

Für die therapeutische Dosierung nach einer Einstiegsphase kann beispielsweise eine Dosierung von 120,0 mg Dimethylfumarat, 87,0 mg des Calciumsalzes des Monoethylfumarats, 5,0 mg des Magnesiumsalzes des Monoethylfumarats und 3,0 mg des Zinksalzes des Monoethylfumarats zur Anwendung kommen.

Die in den erfindungsgemäßen Zubereitungen enthaltenen Fumarsäurederivate werden beispielsweise dadurch erhalten, daß man eine Verbindung der folgenden Formel

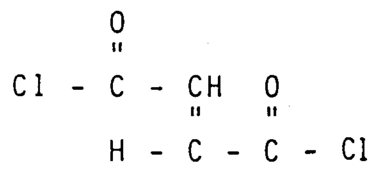

a) mit 2 Mol Alkylalkohol (ROH) zum Diester kondensiert und anschließend kontrolliert zum Monoester hydrolysiert, oder

b) mit 1 Mol eines entsprechenden Alkylalkohols (ROH) kondensiert und das erhaltene Monosäurechlorid zur Säure hydrolysiert, oder

c) Fumarsäure direkt mit 2 Mol Alkylalkohol (ROH) gemäß Anspruch 1 zu einem Diester kondensiert und anschließend kontrolliert zum Monoester hydrolysiert, oder

d) Maleinsäure oder Maleinsäureanhydrid direkt mit 1 - 2 Mol des entsprechenden Alkylalkohols (ROH) nach Anspruch 1 zu einem Mono- oder Diester kondensiert und anschließend katalytisch zum entsprechenden Fumarsäurederivat isomerisiert.

Die Salze der Fumarsäure-monoalkylester können dadurch erhalten werden, daß man eine Verbindung der allgemeinen Formel

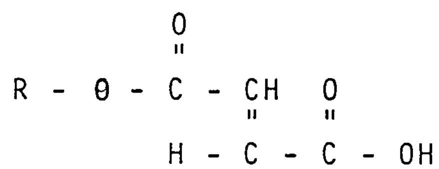,

in der R eine $C_1$-$C_5$- Alkylgruppe bedeutet, mit einem halben Mol Ca-, Mg- oder Zn-Hydroxid oder -Oxid in Toluol zur Umsetzung bringt und das während der Reaktion gebildete Wasser azeotrop entfernt.

Beispiel 1

Herstellung von Filmtabletten mit magenresistentem Ueberzug enthaltend 210 mg Monoethylfumarat-Ca-Salz entsprechend 150 mg Fumarsäure:

21,000 kg Monoethylfumarat-Calciumsalz werden zerkleinert, gemischt und mittels eines Siebes 800 unter entsprechenden Vorsichtsmassnahmen (Atemmaske, Handschuhe, Schutzanzug etc.) homogenisiert. Anschliessend wird ein Hilfsstoffgemisch folgender Zusammensetzung hergestellt: 20,000 kg Stärkederivat (STA-RX 1500 $^{R}$), 2,000 kg mikrokristalline Cellulose (Avicel PH 101 $^{R}$), 0,600 kg Polyvinylpyrolidone (PVP, Kollidon $^{R}$ 25), 4,000 kg Primogel $^{R}$, 0,300 kg kolloidaler Kieselsäure (Aerosil $^{R}$). Das gesamte Pulvergemisch wird mit dem Wirkstoff versetzt und mittels eines Siebes 200 homogenisiert und mit einer 2%igen wässrigen Lösung von Polyvinylpyrolidon (Kollidon $^{R}$K 30) auf übliche Weise zu einem Bindemittelgranulat verarbeitet und in trockenem Zustand mit der äusseren Phase gemischt. Diese besteht aus 2,000 kg eines sogenannten FST-Komplexes, enthaltend 80 % Talk, 10 % Kieselsäure und 10 % Magnesiumstearat. Es wird anschliessend auf übliche Weise zu gewölbten Tabletten von 500 mg Gewicht und 11,5 mm Durchmesser gepresst. Anstelle dieser klassischen Tablettiermethoden können auch andere Methoden zur Herstellung von Tabletten angewendet werden, die Direkttablettierung sowie feste Dispersionen nach der Schmelzmethode und der Sprühtrocknungsmethode.

Magenresistenz:

Es wird eine Lösung von 2,250 kg Hydroxypropylmethylcellulosephthalat (HPMCP, Pharmacoat HP 50 R) in einem Lösungsmittelgemisch von 2,50 l demineralisiertem Wasser, 13,00 l Aceton Ph.Helv. VII und 13,00 l Ethanol 94 Gew.-% gelöst und die Lösung mit 0,240 kg Rizinusöl (Ph. Eur. II) versetzt. Die Lösung wird im Dragierkessel auf traditionelle Weise in Portionen auf die Tablettenkerne aufgeleert oder aufgesprüht bzw. in einem Wirbelschichtapparat entsprechender Konstruktion aufgetragen.

Nach entsprechender Trocknung wird anschliessend der Filmüberzug angebracht. Dieser setzt sich zusammen aus einer Lösung von Eudragit R E 12,5 % 4,800 kg, Farblack ZLT 2 blau (Siegle) 0,210 kg, Titan (VI)-oxyd Kronos RN 56 0,520 kg, Talk (Ph. Eur.) 0,340 kg und Polyethylenglycol 6000 Ph.Helv. VII 0,120 kg. in einem Lösungsgemisch von 8,200 kg 2-Propanol, Ph. Helv. VII, 0,060 kg Glycerintriacetat und 0,200 kg Aqua demineralisata. Nach homogener Verteilung im Dragierkessel oder Wirbelschichtbett wird getrocknet und auf übliche Weise poliert.

Beispiel 2

Herstellung von magenresistenten Kapseln enthaltend 86,5 mg Monoethylfumarat Ca-Salz und 110,0 mg Dimethylfumarat entsprechend insgesamt 150 mg Fumarsäure

8,650 kg Monoethylfumarat Ca-Salz und 11,000 kg Dimethylfumarat werden mit einem Gemisch bestehend aus 15,000 kg Stärke, 6,000 kg Lactose Ph. Helv. VII, 2,000 kg mikrokristalliner Cellulose (Avicel R), 1,000 kg Polyvinylpyrolidon (Kollidon R 25) und 4,000 kg Primogel R intensiv gemischt und mittels eines Siebes 800, unter Beachtung entsprechender Schutzmassnahmen (Atemmaske, Handschuhe, Schutzanzug etc.), homogenisiert. Das gesamte Pulvergemisch wird mit einer 2%igen wässrigen Lösung von Polyvinylpyrolidon (Kollidon R 25) auf übliche Weise zu einem Bindemittelgranulat verarbeitet und in getrocknetem Zustand mit der äusseren Phase gemischt. Diese besteht aus 0,350 kg kolloidaler Kieselsäure (Aerosil R), 0,500 kg Mg-Stearat und 1,500 kg Talkum Ph. Helv. VII. Das homogene Gemisch wird anschliessend in entsprechende Kapseln in Portionen von 500,0 mg abgefüllt, welche abschliessend auf übliche Weise mit einem magenresistenten Ueberzug, bestehend aus Hydroxypropylmethylcellulosestearat und Rizinusöl als Weichmacher, versehen werden. Die Abfüllung kann ebenfalls anstelle von Hartgelatinekapseln in entsprechende magenresistente Kapseln, bestehend aus einem Gemisch von Celluloseacetatphthalat (CAP) und Hydroxypropylethylcellulosephthalat (HPMCP) erfolgen.

Beispiel 3

Herstellung von magenresistenten Kapseln, enthaltend 203,0 mg Monoethylfumarat Ca-Salz sowie 5,0 mg Monoethylfumarat Mg-Salz und 3,0 mg Monoethylfumarat Zn-Salz entsprechend insgesamt 150 mg Fumarsäure

20,300 kg Monoethylfumarat Ca-Salz sowie 0,500 kg Monoethylfumarat Mg-Salz und 0,300 kg Monoethylfumarat Zn-Salz werden zerkleinert, intensiv gemischt und unter entsprechenden Schutzmassnahmen (Atemmaske, Handschuhe, Schutzanzug etc.) mittels eines Siebes 800 homogenisiert. Diesem Wirkstoffgemisch wird ein homogenes Pulvergemisch folgender Zusammensetzung untergemischt: sprühgetrocknete Lactose 12,900 kg, kolloidale Kieselsäure 1,000 kg, mikrokristalline Cellulose (Avicel R) 2,000 kg, Magnesiumstearat (Ph. Helv. VII) 1,000 kg und Talk (Ph. Helv. VII) 2,000 kg. Das gesamte Pulvergemisch wird nochmals mittels eines Siebes 200 homogenisiert und anschliessend in Hartgelatine-Steckkapseln zu 400 mg Nettogewicht eingefüllt und verschlossen. Das Ueberziehen mit einem magenresistenen Ueberzug erfolgt wie in Beispiel 2.

Beispiel 4

Herstellung von magenresistenten Tabletten, enthaltend 87,0 mg Monoethylfumarat Ca-Salz, 120,0 mg Dimethylfumarat, 5,0 mg Monoethylfumarat Mg-Salz und 3,0 mg Monoethylfumarat Zn-Salz entsprechend 164 mg Fumarsäure ("Forte"-Tabletten)

12,000 kg Fumarsäuredimethylester, 8,700 kg Monoethylfumarat Ca-Salz, 0,500 kg Monoethylfumarat Mg-Salz und 0,300 kg Monoethylfumarat Zn-Salz werden zerkleinert, intensiv gemischt und mittels eines Siebes 800 homogenisiert, unter Beachtung entsprechender Schutzmassnahmen (Atemmaske, Handschuhe,

Schutzanzug etc.). Es wird ein Hilfsstoffgemisch folgender Zusammensetzung, auf ähnliche Weise wie unter Beispiel 1 aufgeführt, hergestellt: Stärkederivat (STA-RX 1500 $^{R}$) 18,000 kg, mikrokristalline Cellulose (Avicel pH 101 $^{R}$) 0,300 kg, Polyvinylpyrolidone (PVP, Kollidon $^{R}$ 120) 0,750 kg, Primogel $^{R}$ 4,000 kg und kolloidale Kieselsäure (Aerosil $^{R}$) 0,250 kg.

Hilfsstoffe und Wirkstoffgemisch werden intensiv gemischt und mittels eines Siebes 200 homogenisiert. Das Ganze wird mit einer 2%igen wässrigen Lösung von Polyvinylpyrolidon (Kollidon $^{R}$ K25) auf übliche Weise zu einem Bindemittelgranulat verarbeitet und in getrocknetem Zustand mit der äusseren Phase gemischt. Diese besteht auf 0,500 kg Mg-Stearat (Ph. Eur.) und 1,500 kg Talk (Ph. Eur. II). Das ganze Granulat wird anschliessend auf übliche Weise zu gewölbten Tabletten von 500 mg Bruttomasse und 11,5 mm Durchmesser gepresst. Anstelle dieser klassischen Tablettiermethode können auch andere Methoden zur Tablettenherstellung angewendet werden, wie Direkttablettierung sowie feste Dispersionen nach der Schmelz- und der Sprühtrocknungsmethode.

Der magensaftresistente Ueberzug kann in einem klassischen Dragierkessel aufgeleert oder aufgesprüht sowie in einer Wirbelschichtapparatur erfolgen. Zur Magenresistenz wird portionenweise eine Lösung von 2,250 kg Hydroxypropylmethylcellulosephthalat (HPMCP, Pharmacoat HP 50 $^{R}$) in einem Gemisch folgender Lösungsmittel aufgelöst: Azeton 13,00 l, Ethanol 94 Gewichtsprozent denaturiert mit 2 % Keton 13,50 l und Aqua demineralisata 2,50 l. Zu der fertigen Lösung wird als Weichmacher Rizinusöl Ph. Eur. 0,240 kg zugegeben und auf übliche Weise in Portionen auf die gewölbten Tablettenkerne aufgetragen.

Filmcoat: Nach beendeter Trocknung wird anschliessend in den gleichen Apparaturen eine Suspension folgender Zusammensetzung als Filmcoat aufgezogen: Talcum Ph. Eur. II 0,340 kg, Titan (VI)-oxyd Cronus RN 56 $^{R}$ 0,400 kg, Farblack L-Rotlack 86237 N 0,324 kg, Eudragit E 12,5 % $^{R}$ 4,800 kg und Polyethylenglycol 6000 pH 11 XI 0,120 kg in einem Lösungsgemisch folgender Zusammensetzung: 2-Propanol DAB 8,170 kg, Aqua demineralisata 0,200 kg und Glycerintriacetat (Triacetin $^{R}$) 0,060 kg.

## Beispiel 5

Herstellung von magenresistenten Filmtabletten enthaltend 67,0 mg Monoethylfumarat Ca-Salz, 30,0 mg Dimethylfumarat, 5,0 mg Monoethylfumarat Mg-Salz und 3,0 mg Monoethylfumarat Zn-Salz entsprechend 75 mg Fumarsäure ("Mite"-Tabletten)

3,000 kg Fumarsäuredimethylester, 6,700 kg Monoethylfumarat Ca-Salz, 0,500 kg Monoethylfumarat Mg-Salz sowie 0,300 kg Monoethylfumarat Zn-Salz werden zerkleinert, intensiv gemischt und mittels eines Siebes 800 homogenisiert. Dabei sollen entsprechende Schutzmassnahmen wie Atemmaske, Handschuhe, Schutzanzug etc. zur Anwendung gelangen. Hierauf wird in einem Gemisch, bestehend aus 30,000 kg Stärkederivat (STA-RX 1500 $^{R}$), 3,000 kg mikrokristalline Cellulose (Avicel pH 101 $^{R}$), 0,750 kg Polyvinylpyrolidone (PVP Kollidon $^{R}$ 25), 4,000 kg Primogel $^{R}$, 0,250 kg kolloidale Kieselsäure (Aerosil $^{R}$) versetzt. Das Wirkstoffgemisch wird homogen untermischt, durch ein Sieb 200 geschlagen und mit einer 2%igen wässrigen Lösung von Polyvinylpyrolidon (K 25) auf übliche Weise zu einem Bindemittelgranulat aufgearbeitet. Dem getrockneten Granulat wird eine Pulvermischung aus folgenden Hilfsstoffen als äussere Phase zugesetzt: 0,500 kg Mg-Stearat Ph. Eur. II und 0,800 kg Talk Ph. Helv. VII.

Die homogene Granulatmischung wird zu gewölbten Tablettenkernen von 500,0 mg Gewicht und 11,5 mm Durchmesser auf übliche Weise komprimiert. Neben den Bindemittelmethoden können ebenfalls andere Tablettiermethoden, gemäss den Beispielen 1 und 4, Verwendung finden.

Das Ueberziehen der Tablettenkerne mit einem magenresistenten Ueberzug sowie mit einem Filmcoat erfolgt sinngemäss wie unter den Beispielen 1 und 4 beschrieben.

Vorzugsweise werden die erfindungsgemäßen Zubereitungen peroral in Form von Tabletten oder Kapseln verabreicht, wobei diese festen Einzeldosis-Arzneiformen vorzugsweise mit einem magenresistenten Überzug versehen sind, der sich nach der Magenpassage im Dünndarmsaft im Dünndarm innerhalb weniger Minuten löst und das aktive Prinzip aus der Arzneiform freisetzt. Zum systemischen Einstieg bzw. Ausstieg ist eine niedrige Dosierung (mite) erforderlich, für die therapeutische Dosierung nach der Einstiegsphase eine höhere Dosierung (forte).

Es wurde festgestellt, daß die erfindungsgemäßen Mischpräparate nach peroraler Verabreichung eine erheblich verbesserte Wirkung gegen die verschiedensten klinischen Erscheinungsformen der Psoriasis , der psoriatischen Arthritis, der Neurodermitis sowie der Enteritis regionalis Crohn (Morbus Crohn) aufweisen.

Da in einer psoriatischen Epidermis die Aktivität der Phospholipase $A_2$ verändert ist, liegt eine mögliche Erklärung des Wirkungsmechanismus der erfindungsgemäßen Mischpräparate darin, daß dieses Enzym durch Calcium-Monoethylfumarat stimuliert wird, wobei Mg- und Zn-Kationen für den Hautstoffwechsel von

Psoriasis-Patienten von großer Wichtigkeit sind.

Gegenstand der Erfindung sind neben oral verabreichbaren Präparaten in Form von Kapseln, Granulaten und Tabletten, für die kutane und transdermale Verabreichung in Form von Salben, Pflastern, Lotionen und Duschmitteln, für die parenterale Verabreichung in Form wäßriger Mikrodispersionen, O/W-Emulsionen oder öliger Lösungen für die rektale Verabreichung als Suppositorien oder Mikroklistiere sowie für die medikamentöse Behandlung von Haaren, Finger- und Zehennägeln.

Therapeutische Behandlung der Psoriasis mit dem Präparat gemäß Beispiel 4 und ihre Ergebnisse

In einer intraindividuellen Verlaufsstudie über ein Jahr wurde die ambulante perorale Behandlung der Psoriasis an insgesamt 24 Patienten erprobt (siehe Tabelle 1). Alle Patienten sprachen dabei vorher auf konventionelle Arzneistoffe und Therapieformen schlecht an, so daß von einer negativen Selektion gesprochen werden kann.

Die Hälfte aller peroral und ambulant behandelten Patienten zeigte objektiv eine wesentliche Besserung, welche meist erst nach mehrwöchiger Behandlung eintrat.

Schwerer objektive Nebenwirkungen, insbesondere Nieren- und Leberfunktionsstörungen oder Blutbildveränderungen, wurden keine festgestellt.

Die akute Toxizität wurden vor der klinischen Prüfung an Mäusen und Ratten peroral untersucht. Die Resultate zeigten eine sehr geringe Toxizität der eingesetzten Fumarsäurederivate (siehe Tabelle 2).

Tabelle 1: Klinische Ergebnisse bei Psoriasisbehandlung

| | Studie I | Studie II |
|---|---|---|
| | n = 13 Patienten<br>Formulierg gemäss Beispiel 4 | n = 11 Patienten<br>Formulierg gemäss Beispiel 4 |
| 1. Dauer der Behandlung | 3 Monate | 1 Jahr |
| 2. Resultate: | | |
| — sehr gut | 4 Patienten | 5 Patienten |
| — gut | 3 Patienten | 1 Patient |
| — unbefriedigend | 4 Patienten | 3 Patienten |
| 3. Therapieabbruch wegen Nebenwirkungen | 2 Patienten | 2 Patienten |

Tabelle 2

| Akute Toxizitätsstudie (oral) | | | | |
|---|---|---|---|---|
| | | Sex of Animals | Zusammensetzung gemäss Beispiel 4 | |
| | | | Mice | Rats |
| $LD_{50}$ (24 hours) | in mg/kg | male<br>female | 5750<br>8200 | 4700<br>4600 |
| $LD_{50}$ (14 days) | in mg/kg | male<br>female | 5600<br>6950 | 4700<br>3900 |
| Lowest Toxical Dose | in mg/kg | male<br>female | 3160<br>3160 | 3160<br>3160 |
| Lowest Lethal Dose | in mg/kg | | 5620 | 4640 |
| Stomach & Intestinal Wall Haemorragic | in mg/kg | | 5600 | none |
| Spleen Inflammatory | | | none | none |
| Oedema Epithelial | | | none | none |

Im folgenden wird die Behandlung von Patienten mit Neurodermitis und Enteritis regionalis Crohn (Morbus Crohn) und ihr therapeutisches Ergebnis beschrieben:

HM - weiblich - 1951

- Krankheit: Enteritis regionalis Crohn
- Dosis: 3 Tabletten/Tag (Formulierung gemäß Beispiel 5)
- Dauer: 7.5.86 - 27.11.86
- Erfolg: Darm seither ruhig ohne Medikation

EL - weiblich - 1919

- Krankheit: Enteritis regionalis Crohn
- Therapiebeginn: 24.3.84
- Dosis: 3 Tabletten/Tag (Formulierung gemäß Beispiel 5)
- Erfolg: Darmblutungen und Geschwüre zur Ruhe gekommen

WCh - weiblich - 1945

Krankheit: Enteritis regionalis Crohn
- Therapiebeginn: 9.4.1988
- Dosis: 3 Tabletten/Tag (Formulierung gemäß Beispiel 5)
- Erfolg: alles ruhiger, aber noch zu früh zum beurteilen

NK - männlich - 1971

- Krankheit: Neurodermitis
- Dauer der Krankheit: 16 Jahre
- Therapiebeginn: 16.12.87
- Dosis: 3 Tabletten/Tag (Formulierung gemäß Beispiel 4)
- Erfolg: sehr gut

DN - weiblich - 1926

- Krankheit: Neurodermitis
- Dauer der Krankheit: 40 Jahre
- Therapiebeginn: 4.9.86

- Dosis: 2 Tabletten/Tag (Formulierung gemäß Beispiel 4)
- Erfolg: gut

AS - weiblich - 1941

- Krankheit: Neurodermitis
- Dauer der Krankheit: 20 Jahre
- Therapiebeginn: 16.12.1987
- Dosis: 6 Tabletten/Tag (Formulierung gemäß Beispiel 5)
- Erfolg: sehr gut

D.E. - weiblich - 1960

- Krankheit: Ichthyosis
- Therapiebeginn: 15.3.1988
- Dosis: 6 Tabletten/Tag (Formulierung gemäß Beispiel 5)
- Erfolg: gut

**Patentansprüche**

**1.** Pharmazeutische Zubereitungen zur Behandlung der Psoriasis, dadurch gekennzeichnet, daß sie eine oder mehrere Verbindungen aus der Gruppe der Calcium-, Magnesium-, Zink- und Eisensalze von Fumarsäure-Monoalkylester der allgemeinen Formel

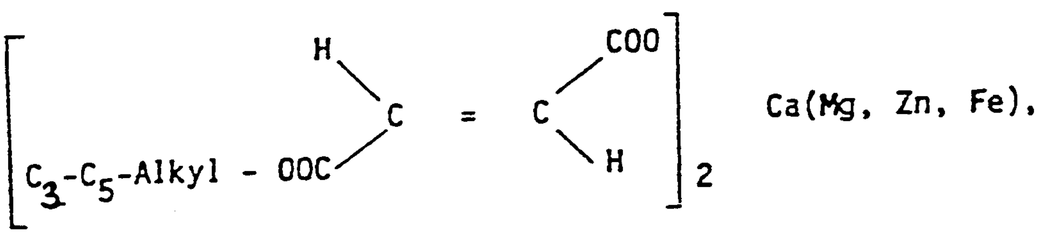

und übliche pharmazeutische Hilfs- und Trägerstoffen enthält.

**2.** Pharmazeutische Zubereitungen zur Behandlung der Psoriasis, dadurch gekennzeichnet, daß sie eine oder mehrere Verbindungen aus der Gruppe der Calcium-, Magnesium-, Zink- und Eisensalze von Fumarsäure-Monoalkylester der allgemeinen Formel

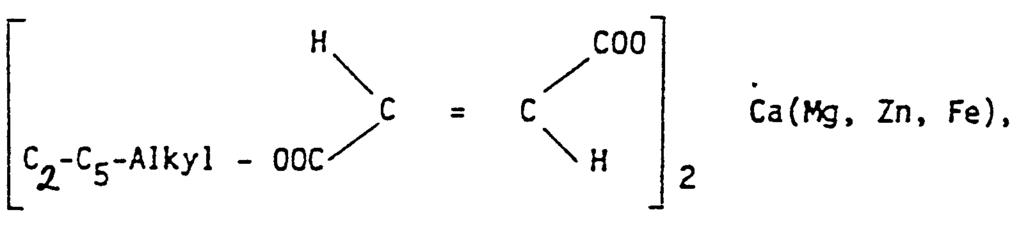

im Gemisch mit Dialkylfumarat der Formel

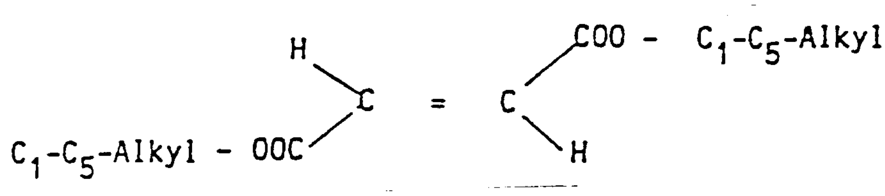

und üblichen pharmazeutischen Hilfs- und Trägerstoffen enthält, wobei die Kombination Fumarsäuremonoethylestersalz und Dimethylfumarat ausgenommen ist.

**3.** Pharmazeutische Zubereitungsform zur oralen Verabreichung in Form von Tabletten oder Kapseln nach Anspruch 1 oder 2, dadurch gekennzeichnet, daß sie das Calciumsalz des Fumarsäure-Monoalkylesters in einer Menge von 100 bis 300 mg enthält, wobei das Gesamtgewicht der Wirkstoffe 100 bis 300 mg beträgt.

**4.** Pharmazeutische Zubereitungsform zur oralen Verabreichung in Form von Tabletten oder Kapseln nach Anspruch 1 oder 2, dadurch gekennzeichnet, daß sie 10 bis 290 Gew.-Teile des Calciumsalzes des Fumarsäure-Monoalkylesters und 290 bis 10 Gew.-Teile Dimethylfumarat enthält, wobei das Gesamtgewicht der Wirkstoffe 100 bis 300 mg beträgt.

**5.** Pharmazeutische Zubereitungsform zur oralen Verabreichung in Form von Tabletten oder Kapseln nach Anspruch 1 oder 2, dadurch gekennzeichnet, daß sie 10 bis 250 Gew.-Teile des Calciumsalzes des Fumarsäure-Monoalkylesters,1 bis 50 Gew.-Teile Dimethylfumarat und 1 bis 50 Gew.-Teile des Zinksalzes des Fumarsäure-Monoalkylesters enthält, wobei das Gesamtgewicht der Wirkstoffe 100 bis 300 mg beträgt.

**6.** Pharmazeutische Zubereitungsform zur oralen Verabreichung in Form von Tabletten oder Kapseln nach Anspruch 1 oder 2, dadurch gekennzeichnet, daß sie 10 bis 250 Gew.-Teile des Calciumsalzes des Fumarsäure-Monoalkylesters,250 bis 10 Gew.-Teile Dimethylfumarat, 1 bis 50 Gew.-Teile des Magnesiumsalzes des Fumarsäure-Monoalkylesters, und 1 bis 50 Gew.-Teile des Zinksalzes des Fumarsäure-Monoalkylesters enthält, wobei das Gesamtgewicht der Wirkstoffe 100 bis 300 mg beträgt.

**7.** Pharmazeutische Zubereitungsform zur oralen Verabreichung nach einem der Ansprüche 1 bis 6, dadurch gekennzeichnet, daß sie mit einem magenresistenten Überzug versehen sind.

**8.** Pharmazeutische Zubereitungen nach Anspruch 1 bis 7 für die perorale Verabreichung in Form von Kapseln, Granulaten und Tabletten, für die kutane und transdermale Verabreichung in Form von Salben, Pflastern, Lotionen und Duschmitteln, für die parenterale Verabreichung in Form wäßriger Mikrodipsersionen, O/W-Emulsionen oder öligen Lösungen, für die rektale Verabreichung als Suppositorien oder Mikroklistiere, sowie für die medikamentöse Behandlung von Haaren, Finger- und Zehennägeln.